# EUROPEAN PATENT SPECIFICATION

(11) **EP 3 423 920 B1**
(45) Date of publication and mention of the grant of the patent: **09.02.2022**
(21) Application number: 17733558.5
(22) Date of filing: 24.02.2017
(51) Int. Cl.: G06F 3/01, G09G 3/00, G06F 3/0488, G06F 3/16, G09B 21/00, A61K 33/22, A61K 9/00, A61P 19/02

(54) **THERAPEUTIC MIXTURES FOR TREATING OSTEOARTHRITIS COMPRISING NANO HEXAGONAL BORON NITRIDE COMPOSITION**
THERAPEUTISCHE MISCHUNGEN ZUR BEHANDLUNG VON OSTEOARTHRITIS MIT NANOHEXAGONALER BORNITRIDZUSAMMENSETZUNG
MÉLANGES THÉRAPEUTIQUES DESTINÉS AU TRAITEMENT DE L'OSTÉOARTHRITE COMPRENANT UNE COMPOSITION DE NITRURE DE BORE HEXAGONAL NANOMÉTRIQUE

(30) Priority: 02.03.2016 TR 201602710; 12.05.2016 TR 201606270
(43) Date of publication of application: 09.01.2019
(73) Proprietor: Bortek Bor Teknolojileri Ve Mekatronik Sanayi Ticaret Anonim Sirketi, Eskisehir (TR)
(72) Inventor: AY, Suleyman, Odunpazari/Eskisehir (TR); AY, Nuran, Eskisehir (TR); GONCU, Yapincak, Odunpazari/Eskisehir (TR)
(74) Representative: Sevinç, Cenk
(86) International application number: PCT/TR2017/000033
(87) International publication number: WO 2017/171672

(56) References cited:
- WO-A2-2015/038088
- DE-A1-102006 056 814
- O. N. ÇELIK ET AL: "Effect of Nano Hexagonal Boron Nitride Lubricant Additives on the Friction and Wear Properties of AISI 4140 Steel", PARTICULATE SCIENCE AND TECHNOLOGY, vol. 31, no. 5, 3 September 2013 (2013-09-03), pages 501-506, XP055395668, US ISSN: 0272-6351, DOI: 10.1080/02726351.2013.779336
- ALPTUG ATILA ET AL: "Study of the boron levels in serum after implantation of different ratios nano-hexagonal boron nitride-hydroxy apatite in rat femurs", MATERIALS SCIENCE AND ENGINEERING C., vol. 58, 11 September 2015 (2015-09-11), pages 1082-1089, XP055395677, CH ISSN: 0928-4931, DOI: 10.1016/j.msec.2015.09.041

## Description

This invention relates to the mixtures employed in the treatment of the osteoarthritis, said mixtures being formed by the materials comprising the biocompatible solutions, lotions, creams, materials that contain chemical oils, materials that contains animal oils, materials that contains vegetable oils, ointments, gels, foams, plasters, compounds for enhancing the skin penetration and pomades which are able to be applied topically to the skin, along with the therapeutic nano hexagonal boron nitride (hBN) composition. When said mixtures are applied to the skin, they penetrate the skin and reach the cartilage and bone ends. The mixture coats the cartilage and fills in the pores in the bone. The nano hBN is a biocompatible solid lubricant and prevents infection. The nano hBN composition lubricates the bone ends to facilitate bone movements, reduces the cartilage loss.

Osteoarthritis is a joint disease involving the destruction or loss of cartilage. The cartilage is a protein-containing material, which serves the function of a pad between the joints of the bones. The osteoarthritis is believed to result from the mechanical stress and the inflammatory processes. The development of the disease includes the loss of cartilage and the effect of this loss on the underlying bone. The extreme states of the disease enormously limit and obstruct a patient's life. The patients with osteoarthritis suffer from depression, incapability, limitations in performance and difficulty in performing the daily activities.

The pills, syrups, creams and lotions are used in the treatment of osteoarthritis. These include the following:
A- Analgesics: These are the analgesics that include acetaminophen, opioids (narcotics) and the atypical opioid called tramadol.
B- Nonsteroidal anti-inflammatory drugs: These are the most commonly employed drugs for relieving the inflammations and the pain associated with inflammation and include aspirin, ibuprofen, naproxen and celecoxib.
C- Corticosteroids: These are very powerful anti-inflammatory agents. These are administered orally in the form of pills or are directly injected into the joint.
D- Lubricant-Hyaluronic acid injection: Hyaluronic acid naturally forms in the joints and serves the function of a lubricant and shock absorber. However, hyaluronic acid is subject to breakdown in the patients with osteoarthritis. The injection of hyaluronic acid may serve the function of a pad in the knee to relieve the pain.

The patents related to the subject matter are also mentioned below:
The patent no. US20100016257 relates to the treatment of osteoarthritis by way of injecting the biodegradable and biocompatible microparticles made from natural and synthetic materials and polymers, which are carried in an aqueous vehicle, into the local area where the osteoarthritis is present.
The patent no. US8765714 relates to the oral or intra-articular formulations of sulphated hyaluronic acid, which are effective in the treatment of osteoarthritis and which do not cause inflammation.
The patent no. US8779006 describes that a pharmaceutical composition comprising sodium bicarbonate and calcium gluconate may be used for the prevention and treatment of osteoarthritis.
The patent no. US6346519 relates to the use of a composition comprising a nitric oxide synthase inhibitor and amino sugars in the treatment of osteoarthritis.
The patent no. CN104474110 describes the treatment of osteoarthritis with a Chinese medical mixture comprising mustard seeds, rehmannia root, millettia, cinnamon, cyathula, ephedra and sichuan. exposed bone. In addition, it features the therapeutic effect for the resulting joint inflammation (arthritis).
The patent no. DE 102006056814 A1 discloses a wound scale preventive composition for maintaining and/or improving healthy skin within a region covered by an article, which comprises boron nitride particles, where the composition leaves a layer of the boron nitride particles that reduces the wound scaling when topically applied on the skin.
The patent no. WO 2015/038088 A2 antifouling additive comprising nano-size hexagonal boron nitride (hBN) , said additive being developed for preventing attachment of various biofouling organisms encountered in the submerged parts of the steel, fiber and wooden ships, in the structures submerged in the sea/water, in any kind of net including the aquaculture nets, in the floats and in the industrial water systems, and being added to the materials such as paints, varnishes, thin films, etc., as well as to the applications of the same.

Osman Nuri Celik et al. have studied on the effect of nano hexagonal boron nitride lubricant additives on the friction and wear properties of AISI 4140 steel. According to the study, nano hexagonal boron nitride particles that were used as an oil additive affected the friction and wear behavior. 14.4% improvement in the friction coefficient and 65% decrease in the wear rate were achieved through the use of the nano hBN as an oil additive.

Alptug Atila et al have studied about boron levels in serum after implantation of different ratios nano-hexagonal boron nitride-hydroxy apatite in rat femurs. In this study, it's demonstrated that neither short-term nor long-term implantation of hBN-HA composite resulted in statistically increased serum boron levels in experimental groups compared to healthy group. In conclusion, this study investigated the implant material produced form hBN-HA for the first time.

The patents and applications mentioned above reduce the pain and inflammation temporarily or provide temporary lubrication in the joints for the treatment of osteoarthritis. The therapy is not long lasting or permanent. The invention is defined by the appended claims.

The mixtures described in the present invention comprise a biocompatible solid lubricant, which simultaneously facilitates the bone movements by way of lubrication, is absorbed by the bone and cartilage, reduces the cartilage loss and prevents infection. The mixtures described in the present invention increase the lubrication while treating the inflammation and provide a long term cure for the region of osteoarthritis. The mixtures described in the present invention, owing to the presence of the solid lubricant, provide a therapy that is much more effective and permanent as compared to the other practices.

With this invention, the mixtures of materials comprising the biocompatible solutions, lotions, creams, ointments, gels, foams, plasters, pomades, compounds for enhancing the skin penetration and chemical, animal and vegetable oils, which are able to be applied topically to the skin, along with the solid lubricant, i.e. nano hexagonal boron nitride (hBN) composition, are used in the treatment of the osteoarthritis. When the mixtures are externally applied to the skin, the nano-size hBN composition penetrates the skin to fill in the smallest pores on the exposed bone and becomes located in the mass of the cartilage. Nano hBN composition is a biocompatible solid lubricant and anti-infective agent. Thus, it improves the lubricating property for the destroyed or lost cartilage and exposed bone. In addition, it features the therapeutic effect for the resulting joint inflammation (arthritis).

According to the present invention, the composition comprising nano hBN with a particle size D50 of less than 200 nanometers is prepared together with the biocompatible vegetable, animal and chemical oils and the compounds for enhancing the skin penetration. The nano hBN composition comprises more than 90% by weight hBN and less than 10% by weight boron oxide. The composition comprising the nano-size hBN is mixed with the biocompatible vegetable, animal and chemical oils and the compounds for enhancing the skin penetration by means of mechanical and/or ultrasonic methods and the mixture is exfoliated. The exfoliated mixture contains less than 5% by weight of the composition that comprises nano hBN.

The exfoliated mixture is used directly as a therapeutic for the treatment of osteoarthritis.

The exfoliated mixture is mixed with a biocompatible solution at a ratio smaller than 20% and is used as a therapeutic solution for treating osteoarthritis.

The exfoliated mixture is mixed with a biocompatible lotion at a ratio smaller than 20% and is used as a therapeutic lotion for treating osteoarthritis.

The exfoliated mixture is mixed with a biocompatible ointment at a ratio smaller than 50% and is used as a therapeutic ointment for treating osteoarthritis.

The exfoliated mixture is mixed with a biocompatible cream at a ratio smaller than 50% and is used as a therapeutic cream for treating osteoarthritis.

The exfoliated mixture is mixed with a biocompatible gel at a ratio smaller than 50% and is used as a therapeutic gel for treating osteoarthritis.

The exfoliated mixture is mixed with a biocompatible plaster at a ratio smaller than 50% and is used as a therapeutic plaster for treating osteoarthritis.

The exfoliated mixture is mixed with a biocompatible pomade at a ratio smaller than 50% and is used as a therapeutic pomade for treating osteoarthritis.

The exfoliated mixture is mixed with a biocompatible foam at a ratio smaller than 50% and is used as a therapeutic foam for treating osteoarthritis.

When the prepared mixtures are applied topically to the skin, they penetrate the skin and reach the joint cartilage region and the bone ends. The nano hBN in the mixtures coats the cartilage and fills in the pores in the bone. The nano hBN is a biocompatible solid lubricant and anti-infective agent. The nano hBN composition lubricates the bone ends to facilitate bone movements, reduces the cartilage loss.

With this invention, it was determined in the studies applied on volunteers that biocompatible solutions, lotions, materials that contains chemical oils, materials that contains animal oils, materials that contains vegetable oils, creams, ointments, gels, foams, plasters, compounds for enhancing the skin penetration, pomades that includes nano hexagonal boron nitride treat the osteoarthritis in the joints when applied topically to skin.

The examples and the results of the tests performed on the volunteers include, but are not limited to, the following:
The therapeutic solution for treating the osteoarthritis was applied to a 60 year old volunteer with difficulty in walking, and after the therapy for one month, the person's walking returned to normal and the person became free of the problem.
The therapeutic ointment for treating the osteoarthritis was applied for 15 days to a volunteer over the age of 50 with difficulty in going up the stairs, and the person became able to easily go up the stairs.

A 65 year old volunteer with difficulty in walking used the therapeutic cream for treating the osteoarthritis for 2 months and the person's walking returned to normal.

## Claims

1. Therapeutic mixture for use in treating the osteoarthritis developing in the knee, hip, shoulder, wrist, ankle, elbow, finger, toe, backbone, **characterized in that** said mixture comprises (a) one or a mixture of chemical oils, animal oils or vegetable oils and (b) compounds for enhancing the skin penetration, and (c) the therapeutic nano hexagonal boron nitride (hBN) composition, wherein said therapeutic mixture is preferably in the form of any biocompatible lotion, solution, cream, ointment, pomade, gel, foam or plaster which can be applied topically to the skin.

2. Therapeutic mixtures for use in treating the osteoarthritis according to Claim 1, **characterized in that** the nano hBN-containing composition in the mixtures comprises more than 90% by weight nano hBN and less than 10% by weight boron oxide.

3. Therapeutic mixtures for use in treating the osteoarthritis according to Claim 1 or 2, **characterized in that** the nano hBN used in the nano hBN composition in the mixtures has a particle size D50 of less than 200 nanometers.

4. Therapeutic mixtures for use in treating the osteoarthritis according to Claim 1 or 2 or 3, **characterized in that** the nano-size hBN-containing composition in the mixtures is mixed with the biocompatible vegetable, animal and chemical oils and the compounds for enhancing the skin penetration by means of mechanical and/or ultrasonic methods and the mixture is exfoliated.

5. Exfoliated mixture for use in the treatment of osteoarthritis according to claim 4 **characterized in that** it contains less than 5% by weight of the composition that comprises nano hBN.

6. Exfoliated mixture for use in the treatment of osteoarthritis according to claim 4 **characterized in that** the exfoliated mixture is able to be used directly.

7. Exfoliated mixture for use in the treatment of osteoarthritis according to claim 4 **characterized in that** the exfoliated mixture is mixed with a biocompatible solution at a ratio smaller than 20%.

8. Exfoliated mixture for use in the treatment of osteoarthritis according to claim 4 **characterized in that** the exfoliated mixture is mixed with a biocompatible lotion at a ratio smaller than 20%.

9. Exfoliated mixture for use in the treatment of osteoarthritis according to claim 4 **characterized in that** the exfoliated mixture is mixed with a biocompatible ointment at a ratio smaller than 50%.

10. Exfoliated mixture for use in the treatment of osteoarthritis according to claim 4 **characterized in that** the exfoliated mixture is mixed with a biocompatible cream at a ratio smaller than 50%.

11. Exfoliated mixture for use in the treatment of osteoarthritis according to claim 4 **characterized in that** the exfoliated mixture is mixed with a biocompatible gel at a ratio smaller than 50%.

12. Exfoliated mixture for use in the treatment of osteoarthritis according to claim 4 **characterized in that** the exfoliated mixture is mixed with a biocompatible plaster at a ratio smaller than 50%.

13. Exfoliated mixture for use in the treatment of osteoarthritis according to claim 4 **characterized in that** the exfoliated mixture is mixed with a biocompatible pomade at a ratio smaller than 50%.

14. Exfoliated mixture for use in the treatment of osteoarthritis according to claim 4 **characterized in that** the exfoliated mixture is mixed with a biocompatible foam at a ratio smaller than 50%.

## Patentansprüche

1. Therapeutische Mischung zur Verwendung in der Behandlung von Osteoarthritis, die sich in dem Knie, der Hüfte, der Schulter, dem Handgelenk, dem Knöchel, dem Ellbogen, dem Finger, dem Zeh und der Wirbelsäule entwickelt, **dadurch gekennzeichnet, dass** die genannte Mischung umfasst (a) ein oder eine Mischung aus chemischen Ölen, tierischen Ölen oder pflanzlichen Ölen und (b) Verbindungen zur Verstärkung der Hautpenetration, und (c) die therapeutische Nano-Hexagonale-Bornitrid (hBN)-Zusammensetzung, wobei die therapeutische Mischung vorzugsweise in Form einer beliebigen biokompatiblen Lotion, Lösung, Creme, Salbe, Pomade, Gel, Schaum oder Pflaster vorliegt, die topisch auf die Haut aufgetragen werden kann.

2. Therapeutische Mischungen zur Verwendung in der Behandlung von Osteoarthritis nach Anspruch 1, **dadurch gekennzeichnet, dass** die nano-hBN-haltige Zusammensetzung in den Mischungen mehr als 90 Gew.-% nano-hBN und weniger als 10 Gew.-% Boroxid umfasst.

3. Therapeutische Mischungen zur Verwendung in der Behandlung der Osteoarthritis nach Anspruch 1 oder 2, **dadurch gekennzeichnet, dass** das in den Mischungen verwendete nano-hBN in der nano-hBN-Zusammensetzung eine Teilchengröße D50 von weniger als 200 Nanometern aufweist.

4. Therapeutische Mischungen zur Verwendung in der Behandlung von Osteoarthritis nach Anspruch 1 oder 2 oder 3, **dadurch gekennzeichnet, dass** die in den Mischungen enthaltene hBN-haltige Zusammensetzung in Nanogröße wird mit den biokompatiblen pflanzlichen, tierischen und chemischen Ölen und den Verbindungen zur Verstärkung der Hautpenetration mittels mechanischer und/oder Ultraschallmethoden gemischt und die Mischung wird exfoliert.

5. Exfolierte Mischung zur Verwendung in der Behandlung von Osteoarthritis nach Anspruch 4, **dadurch gekennzeichnet, dass** diese weniger als 5 Gew.-% der Zusammensetzung enthält, die nano hBN umfasst.

6. Exfolierte Mischung zur Verwendung in der Behandlung von Osteoarthritis nach Anspruch 4, **dadurch gekennzeichnet, dass** die exfolierte Mischung direkt verwendet werden kann.

7. Exfolierte Mischung zur Verwendung in der Behandlung von Osteoarthritis nach Anspruch 4, **dadurch gekennzeichnet, dass** die exfolierte Mischung mit einer biokompatiblen Lösung in einem Verhältnis von weniger als 20% gemischt wird.

8. Exfolierte Mischung zur Verwendung in der Behandlung von Osteoarthritis nach Anspruch 4, **dadurch gekennzeichnet, dass** die exfolierte Mischung mit einer biokompatiblen Lotion in einem Verhältnis von weniger als 20% gemischt wird.

9. Exfolierte Mischung zur Verwendung in der Behandlung von Osteoarthritis nach Anspruch 4, **dadurch gekennzeichnet, dass** die exfolierte Mischung mit einer biokompatiblen Salbe in einem Verhältnis von weniger als 50% gemischt wird.

10. Exfolierte Mischung zur Verwendung in der Behandlung von Osteoarthritis nach Anspruch 4, **dadurch gekennzeichnet, dass** die exfolierte Mischung mit einer biokompatiblen Creme in einem Verhältnis von weniger als 50% gemischt ist.

11. Exfolierte Mischung zur Verwendung in der Behandlung von Osteoarthritis nach Anspruch 4, **dadurch gekennzeichnet, dass** die exfolierte Mischung mit einer biokompatiblen Gel in einem Verhältnis von weniger als 50% gemischt ist.

12. Exfolierte Mischung zur Verwendung in der Behandlung von Osteoarthritis nach Anspruch 4, **dadurch gekennzeichnet, dass** die exfolierte Mischung mit einer biokompatiblen Pflaster in einem Verhältnis von weniger als 50% gemischt ist.

13. Exfolierte Mischung zur Verwendung in der Behandlung von Osteoarthritis nach Anspruch 4, **dadurch gekennzeichnet, dass** die exfolierte Mischung mit einer biokompatiblen Pomade in einem Verhältnis von weniger als 50% gemischt ist.

14. Exfolierte Mischung zur Verwendung in der Behandlung von Osteoarthritis nach Anspruch 4, **dadurch gekennzeichnet, dass** die exfolierte Mischung mit einer biokompatiblen Schaum in einem Verhältnis von weniger als 50% gemischt ist.

## Revendications

1. Mélange thérapeutique pour être utilisé dans le traitement de l'arthrose qui se développe dans le genou, la hanche, l'épaule, le poignet, la cheville, le coude, le doigt, l'orteil, la colonne vertébrale, **caractérisé en ce que** ledit mélange comprend (a) une ou un mélange d'huiles chimiques, d'huiles animales ou d'huiles végétales et (b) des composés afin d'améliorer la pénétration cutanée, et (c) la composition thérapeutique de nano-nitrure de bore hexagonal (hBN), dans laquelle ledit mélange thérapeutique est de préférence sous la forme de toute lotion, solution, crème, onguent, pommade, gel, mousse ou plâtre biocompatible qui peut être appliqué localement sur la peau.

2. Mélanges thérapeutiques pour être utilisés dans le traitement de l'arthrose selon la revendication 1, **caractérisés en ce que** la composition contenant de nano hBN dans les mélanges comprend plus de 90% en poids de nano hBN et moins de 10% en poids d'oxyde de bore.

3. Mélanges thérapeutiques pour être utilisés dans le traitement de l'arthrose selon la Revendication 1 ou 2, **caractérisés en ce que** le nano hBN utilisé dans la composition de nano hBN dans les mélanges a une granulométrie D50 inférieure à 200 nanomètres.

4. Mélanges thérapeutiques pour être utilisés dans le traitement de l'arthrose selon la revendication 1 ou 2 ou 3, **caractérisés en ce que** la composition nanométrique contenant du hBN dans les mélanges est mélangée avec les huiles végétales, animales et chimiques biocompatibles et les composés afin d'améliorer la pénétration cutanée au moyen de méthodes mécaniques et/ou ultrasoniques et le mélange est exfolié.

5. Mélange exfolié pour être utilisé dans le traitement de l'arthrose selon la revendication 4, **caractérisé en ce qu'**il contient moins de 5% en poids de la composition comprenant du nano hBN.

6. Mélange exfolié pour être utilisé dans le traitement de l'arthrose selon la revendication 4, **caractérisé en ce que** le mélange exfolié puisse être directement utilisable.

7. Mélange exfolié pour être utilisé dans le traitement de l'arthrose selon la revendication 4, **caractérisé en ce que** le mélange exfolié est mélangé avec une solution biocompatible à un ratio inférieur à 20%.

8. Mélange exfolié pour être utilisé dans le traitement de l'arthrose selon la revendication 4, **caractérisé en ce que** le mélange exfolié est mélangé avec une lotion biocompatible à un ratio inférieur à 20%.

9. Mélange exfolié pour être utilisé dans le traitement de l'arthrose selon la revendication 4, **caractérisé en ce que** le mélange exfolié est mélangé avec un onguent biocompatible à un ratio inférieur à 50%.

10. Mélange exfolié pour être utilisé dans le traitement de l'arthrose selon la revendication 4, **caractérisé en ce que** le mélange exfolié est mélangé avec une crème biocompatible à un ratio inférieur à 50%.

11. Mélange exfolié pour être utilisé dans le traitement de l'arthrose selon la revendication 4, **caractérisé en ce que** le mélange exfolié est mélangé avec un gel biocompatible à un ratio inférieur à 50%.

12. Mélange exfolié pour être utilisé dans le traitement de l'arthrose selon la revendication 4, **caractérisé en ce que** le mélange exfolié est mélangé avec un plâtre biocompatible à un ratio inférieur à 50%.

13. Mélange exfolié pour être utilisé dans le traitement de l'arthrose selon la revendication 4, **caractérisé en ce que** le mélange exfolié est mélangé avec une pommade biocompatible à un ratio inférieur à 50%.

14. Mélange exfolié pour être utilisé dans le traitement de l'arthrose selon la revendication 4, **caractérisé en ce que** le mélange exfolié est mélangé avec une mousse biocompatible à un ratio inférieur à 50%.
